# EUROPÄISCHE PATENTANMELDUNG

(11) **EP 1 782 755 A2**
(43) Veröffentlichungstag der Anmeldung: **09.05.2007**
(21) Anmeldenummer: 07001054.1
(22) Anmeldetag: 10.06.2005
(51) Int. Cl.: A61C 13/08, A61C 13/00

(54) **Verfahren und Vorrichtung zur Herstellung von Zahnersatz**

(30) Priorität: 10.06.2004 DE 202004009128 U; 23.06.2004 DE 202004009900 U
(62) Teilanmeldung aus: 05759210.7
(71) Anmelder: Willytec GmbH, 82166 Gräfelfing (DE)
(72) Erfinder: Weber, Gerhard, 86932 Pürgen (DE); Holzner, Stephan, 80269 Hohenschäftlarn (DE)
(74) Vertreter: Grünecker, Kinkeldey, Stockmair & Schwanhäusser Anwaltssozietät

(57) **Zusammenfassung**

Die Erfindung betrifft Verfahren und Vorrichtungen zur Herstellung von Zahnersatzteilen, mit digitaler Erfassung von Oberflächendaten eines Restzahnbereichs, dreidimensionaler Gestaltung wenigstens eines Formteils, insbesondere eines Zahnersatzteils für den Restzahnbereich unter Einbeziehung der erfassten Oberflächendaten des Restzahnbereichs und Erstellung entsprechen der Formdaten, und teilweiser oder kompletter Herstellung des wenigstens einen Zahnersatzteiles auf der Basis der Oberflächendaten und Formdaten, wobei einer oder mehrere der nachfolgenden Aspekte berücksichtigt wird: dass eine dreidimensionale Gestaltung von Stegkonstruktionen zwischen Zahnpfeilern und Erstellung ent sprechender Formdaten erfolgt, dass Oberflächendaten eines dreidimensionalen Bissabdruckes, einseitig, an zwei Seiten (beidseitig) oder mehrseitig erfasst werden, dass die Oberflächendaten graphisch dargestellt werden, dass an der graphischen Darstellung der Oberflächendaten wenigstens eine Markierung angebracht wird, und dass Formdaten für ein Zahnersatzteil basierend auf den Oberflächendaten unter Einbeziehung der Markierung in der graphischen Darstellung der Oberflächendaten konstruiert werden, dass Primärkronen mittels 3+1 oder angestellter Achsenfrästechnik gefertigt werden, dass durch "Bergauf/Bergabfräsen" eine optimierte Ausrichtung eines Fräsrohlings zu dem zu fräsenden Teil erfolgt, und/oder dass zum Scannen von Modellierwachsen ein Modellierwachs mit mindestens 1/3 bis 4/5 Gewichtsanteilen von Zirkonoxidpulver verwendet wird

## Beschreibung

Die Erfindung betrifft Verfahren und Vorrichtungen zur Herstellung von Zahnersatz insbesondere auf Basis von CAD/CAM-Technologien.

Die vorliegende Erfindung hat und erreicht das Ziel, die bestehende Technik zu verbessern. Weiterhin schafft die vorliegende Erfindung in vorteilhafter Weise kostengünstigere und einfache Alternativen zu den Ausführungen des Standes der Technik.

Dazu werden entsprechend individuellen Aspekten der Erfindung einzeln oder kombiniert umsetzbare Verfahren und Vorrichtungen geschaffen, wie sie in den jeweiligen unabhängigen Ansprüchen angegeben sind. Vorteilhafte und bevorzugte Weiterbildungen ergeben sich aus den jeweils abhängigen Ansprüchen.

Insbesondere schafft die Erfindung Verfahren und Vorrichtungen zur Herstellung von Zahnersatzteilen, mit digitaler Erfassung von Oberflächendaten eines Restzahnbereichs, dreidimensionaler Gestaltung wenigstens eines Formteils, insbesondere eines Zahnersatzteils für den Restzahnbereich unter Einbeziehung der erfassten Oberflächendaten des Restzahnbereichs und Erstellung entsprechender Formdaten, und teilweiser oder kompletter Herstellung des wenigstens einen Zahnersatzteiles auf der Basis der Oberflächendaten und Formdaten, wobei einer oder mehrere der nachfolgenden Aspekte berücksichtigt wird:
- dass im Rahmen der dreidimensionalen Gestaltung des wenigstens einen Formteils eine dreidimensionale Gestaltung von Stegkonstruktionen zwischen Zahnpfeilern und Erstellung entsprechender Formdaten erfolgt, und dass die Formdaten der dreidimensionalen Gestaltung der Stegkonstruktionen zwischen Zahnpfeilern zur Herstellung des Zahnersatzteiles an eine Fertigungsvorrichtung zur teilweisen oder kompletten Herstellung weitergeleitet werden,
- dass Oberflächendaten eines dreidimensionalen Bissabdruckes, der durch Einbringen eines verformbaren Werkstoffes zwischen Ober- und Unterkiefer eines Patienten während des Aufeinanderpressens der Zähne erzeugt wurde, einseitig, an zwei Seiten (beidseitig) oder mehrseitig erfasst werden, dass sowohl die Oberflächendaten des Restzahnbereichs als auch die Oberflächendaten des Bissabdruckes dargestellt werden, wobei in einem Schritt die Oberflächendaten des Restzahnbereichs eines Gipsmodells vermessen werden, in einem weiteren Schritt das Gipsmodell in eine definierte Relativlage zur Messvorrichtung gebracht wird, der Bissabdruck anschließend auf das Gipsmodell gelegt wird und das gesamte Modell zusammen mit dem Gipsabdruck vermessen wird, in einem weiteren Schritt die räumliche Lage der Daten des Gipsmodells und der Daten des Bissabdrucks zueinander auf einem Bildschirm dargestellt und überprüft werden, und in einem weiteren Schritt das Zahnersatzteil mit Hilfe der Darstellungen des Gipsmodells und des Bissabdruckes gestaltet wird,
- dass ein Gesamtformbauteil aus einer Kombination und/oder Verschmelzung von Geometriedaten wenigstens eines vordefinierten dreidimensionalen Körpers mit Formdaten von zumindest einem dreidimensional gestalteten Zahnersatzteil geschaffen wird,
- dass bei der digitalen Erfassung von Oberflächendaten eines Restzahnbereichs auch Daten betreffend die räumliche Lage und Form wenigstens eines Implantatpfostens erfasst werden, dass aus einer Datenbank ein abgespeichertes für die Position des Zahns, für den der Implantatpfosten steht, passendes Abutment ausgewählt wird, dass dieses Abutment in einer Darstellung des Restzahnbereiches mit dem Implantatpfosten auf letzteren aufgesetzt und an den Restzahnbereich angepasst,
- dass auf einen Implantatspfosten ein gewünschtes Abutment mit Wachs modelliert wird, dass dieses Wachsmodell in einem gesonderten Scanvorgang datenmäßig erfasst wird, und dass eingescannte oder vorbekannte Geometriedaten des Implantatpfostens mit dem Oberflächendatensatz des Wachsmodells so zusammengeführt werden, dass sich ein kompletter Formdatensatz eines Abutments mit zusammengefügter Ober- und Unterseite ergibt,
- dass die Oberflächendaten graphisch dargestellt werden, dass an der graphischen Darstellung der Oberflächendaten wenigstens eine Markierung angebracht wird, und dass Formdaten für ein Zahnersatzteil basierend auf den Oberflächendaten unter Einbeziehung der Markierung in der graphischen Darstellung der Oberflächendaten konstruiert werden,
- dass nach einer ersten Vermessung auf dem Gipsmodell per Wachs grobe Aufstellungen gemacht werden, dass die groben Aufstellungen in einem zweiten Scandurchgang vermessen werden, und dass die zwei Scans hochgenau zueinander korreliert werden,
- dass während einer Modellation oder Modellierung an einem Bildschirm zur Erkennung von Veränderungen der Wandstärken die lokalen Wandstärken der Restauration am Bildschirm in farblichen Abstufungen gekennzeichnet oder teilweise transparent dargestellt werden,
- dass eine Kavität gescannt wird, dass anschließend eine Kantendetektion durchgeführt wird, dass danach ein Inlay im Modell mit Wachs modelliert und in diesem Zustand nochmals gescannt wird, dass die beiden Messdatensätze per 3D-Matching überlagert werden, und dass abschließend ein Differenzdatensatz errechnet wird,
- dass eine Kavität gescannt wird, dass danach ein Inlay im Modell mit Wachs modelliert und in diesem Zustand nochmals gescannt wird, dass über ein kombiniertes Verfahren der "Korrelation" in Form von Übereinanderlegung von zwei Scans und der "Abstandsdetektion" der zwei Scans diejenigen Flächen/Punkte herausgefiltert werden, welche für ein Inlay relevant sind, und dass abschließend ein Differenzdatensatz errechnet wird,
- dass ein Verfahren zur Volumendifferenzbildung angewandt wird,
- dass in einem ersten Schritt mittels Einscannen einer Gipsmodellsituation die räumliche Lage von Implantatspfosten oder Implantatsschrauben bestimmt wird, dass in einem zweiten Schritt die Geometriedaten der Implantatspfosten, insbesondere gemäß Herstellerangaben importiert werden, dass die importierten Daten mit den bei der 3D-Vermessung ermittelten Daten entweder gematcht und am Bildschirm angezeigt werden, und dass in einem nächsten Schritt Kronen- oder Brückenkonstruktionen auf Basis der vorangegangenen Schritte am Bildschirm konstruiert und an die CNC-Maschine zur Fertigung übermittelt werden,
- dass zur Ausgestaltung von Primärkronen an deren Datensatz eine geometrisch vorhandene und auf einem Bildschirm sichtbare Friktionslinie selektiert wird, dass dann eine Stelle oder ein Punkt der Friktionslinie markieren wird, dass ausgehend von diesem Punkt ein Wirkungsbereich gewählt wird, also die Breite einer zu verändernden Stelle, dass im nächsten Schritt die Friktionslinie an diesem Punkt über den gewählten Wirkungsbereich nach oben, unten, innen oder außen gezogen wird, und dass sich daraus sich eine neue Topographie der Primärkrone unter automatischer Beibehaltung der Parallelität zur Einschubrichtung oder unter Beibehaltung des Konuswinkels ergibt,
- dass Primärkronen mittels 3+1 oder angestellter Achsenfrästechnik gefertigt werden,
- dass durch "Bergauf-/Bergabfräsen" eine optimierte Ausrichtung eines Fräsrohlings zu dem zu fräsenden Teil erfolgt, und/oder
- dass zum Scannen von Modellierwachsen ein Modellierwachs mit mindestens 1/3 bis 4/5 Gewichtsanteilen von Zirkonoxidpulver verwendet wird.

Eine Vorrichtung zur Herstellung von Zahnersatzteilen enthält Einrichtungen zur digitalen Erfassung von Oberflächendaten eines Restzahnbereichs, Einrichtungen zur dreidimensionalen Gestaltung wenigstens eines Formteils, insbesondere eines Zahnersatzteils für den Restzahnbereich unter Einbeziehung der erfassten Oberflächendaten des Restzahnbereichs und Erstellung entsprechender Formdaten, und Einrichtungen zur teilweisen oder kompletten Herstellung des wenigstens einen Zahnersatzteiles auf der Basis der Oberflächendaten und Formdaten, sowie Einrichtungen zur Durchführung jeglicher der vorerläuterten Verfahren, einschließlich Datenverarbeitungseinrichtungen, Datenspeichereinrichtungen, Bildschirmeinrichtungen, Eingabe- und Ausgabeeinrichtungen sowie Datenübertragungseinrichtungen und Materialbearbeitungseinrichtungen vorgesehen sind, zur Durchführung der Verfahren gemäß jeglichem der Ansprüche 1 bis 70 ausgelegt und funktional miteinander gekoppelt sind.

Die Erfindung wird anhand von Ausführungsbeispielen nachfolgend unter Bezugnahme auf die Zeichnungen näher erläutert.

Anhand der nachfolgend beschriebenen und in den Zeichnungen dargestellten Ausführungs- und Anwendungsbeispiele wird die Erfindung lediglich exemplarisch näher erläutert. Verfahrens- und Vorrichtungsmerkmale ergeben sich jeweils analog auch aus Vorrichtungs- bzw. Verfahrensbeschreibungen.

Gleiche Bezugszeichen in den einzelnen Figuren und Abbildungen der Zeichnung bezeichnen gleiche oder ähnliche oder gleich oder ähnlich wirkende Komponenten. Anhand der Darstellungen in der Zeichnung werden auch solche Merkmale deutlich, die nicht mit Bezugszeichen versehen sind, unabhängig davon, ob solche Merkmale nachfolgend beschrieben sind oder nicht. Andererseits sind auch Merkmale, die in der vorliegenden Beschreibung enthalten, aber nicht in der Zeichnung sichtbar oder dargestellt sind, ohne weiteres für einen Fachmann verständlich.

Einzelne Merkmale, die im Zusammenhang mit konkreten Ausführungsbeispielen angeben und/oder dargestellt sind, sind nicht auf diese Ausfilhrungsbeispiele oder die Kombination mit den übrigen Merkmalen dieser Ausftthrungsbeispiele beschränkt, sondern können im Rahmen des technisch Möglichen, mit jeglichen anderen Varianten, auch wenn sie in den vorliegenden Unterlagen nicht gesondert behandelt sind, kombiniert werden.

Basierend auf dem Stand der Technik bisheriger Schutzrechtsanmeldungen der Firma Willytec GmbH wurden Erweiterungen der technischen Anwendungsmöglichkeiten der Technologien entwickelt, wie nachfolgend für einzelne Aspekte genauer erläutert wird. Ausgangsbasis der Erfindung ist jeweils, dass dreidimensionale Daten eines menschlichen Kiefers mit präparierten Zahnstümpfen vorliegen.

### 1. Stegkonstruktionen

Bei diesem Aspekt der Erfindung handelt es sich um eine Verbesserung bisher in der Zahntechnik üblicher so genannter Stegkonstruktionen mittels CAD/CAM Technologie:

Als Verbindungselemente zwischen den Zahnpfeilern werden von einer Software in dreidimensionale Daten eines menschlichen Kiefers mit präparierten Zahnstümpfen rechtwinklig oder trapezförmig geformte Stege eingefügt. Die Überkronung der Zahnpfeiler wird in mehreren Schritten durchgeführt.

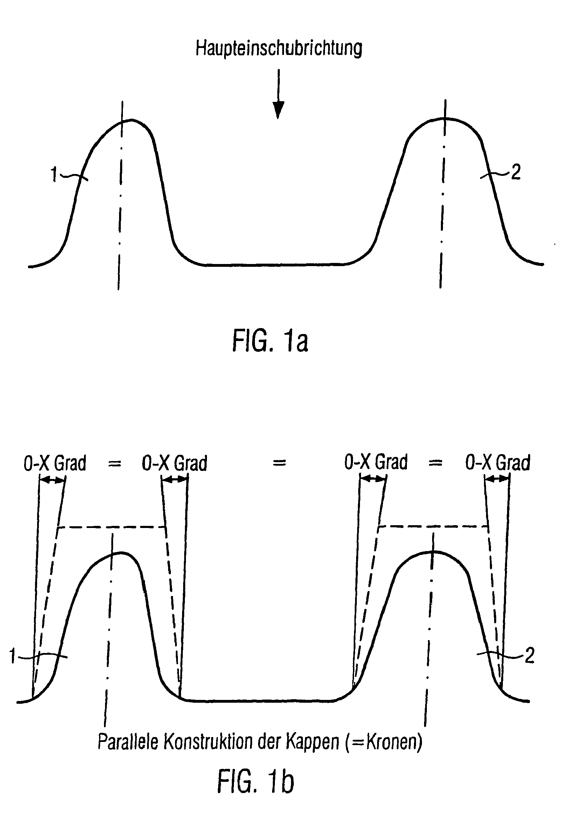

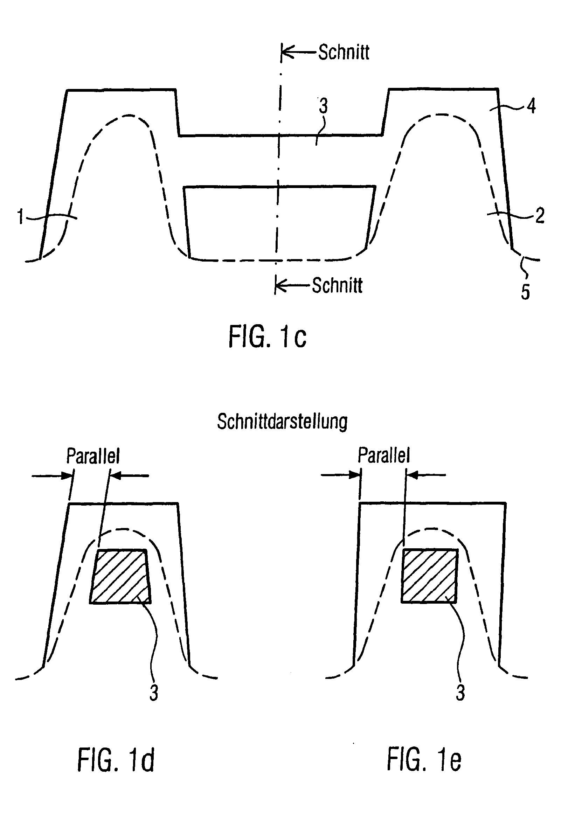

Zunächst wird mittels Software eine Haupteinschubrichtung (Fig. 1a) festgelegt. Dementsprechend werden mittels der Software bereits parallel oder in gleicher Winkellage verlaufende Kronenaußenflächen erzeugt (Fig. 1b). Die Außenflächen aller betroffenen Kronen sind zueinander entweder parallel ausgerichtet, oder die definierten Kronenwinkel sind gleich bezogen auf die Haupteinschubrichtung (Fig. 1a). Nachdem also die Haupteinschubrichtung per Software festgelegt wurde, kann der Steg zwischen den Kronenpfeilern automatisch so eingefügt werden, dass auch dessen Außenflächen parallel zur Haupteinschubrichtung ausgerichtet sind (Fig. 1c, 1d, 1e). In den Figuren 1d und 1e sind Beispiele für eine parallele oder winklige Ausformung des Steges gezeigt. Die Flächen können also auch in üblichen konischen Winkellagen (in der Regel 2°) ausgebildet sein

Somit ist die gesamte Situation parallelwandig bzw. konisch ausgerichtet und kann entsprechend hochpräzise per CNC-Maschine gefräst werden. Die Werkstücke können mittels CNC-Technologie aus verschiedensten Materialien, insbesondere Zirkonoxidkeramik hergestellt werden.

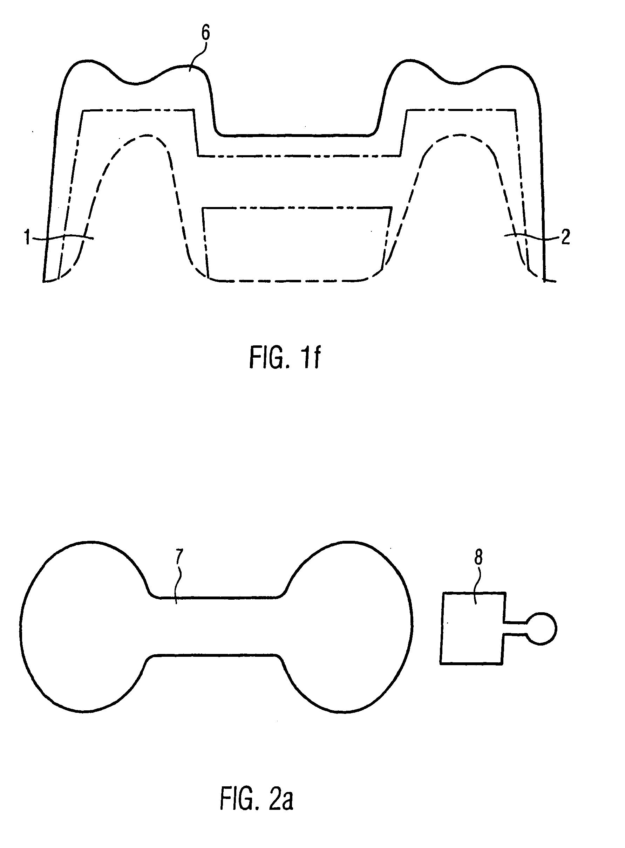

Die vorliegenden Außendaten dieser Stegkonstruktion können wiederum als Innendaten für die Sekundär-/Tertiärkonstruktion weiterverarbeitet werden (unter Addition eines Passmaßes), so dass mittels CNC-Frästechnik eine perfekte, erstmals normierbare Passung entsteht. Die Sekundär- oder Tertiärkonstruktion ist in der Regel für den Patienten abnehmbar gestaltet (Fig. 1f).

### 2. Einblendung des Gegenbisses in eine Modellationssoftware

Bei diesem Aspekt der Erfindung handelt es sich um eine Verbesserung bei der Modellierung von Zahnersatz. Dazu ist die Darstellung des Gegenbisses bzw. gegenüberliegenden Kiefers (mittels Computer) von Vorteil.

Ein von Fachleuten so bezeichnetes Bissregistrat (zentrisch / funktional) wird nach der Ermittlung der Kieferdaten für einen zweiten Messgang auf die bereits vermessene Modellsituation gesetzt. In diesem zweiten Messgang wird dieses Bissregistrat mitvermessen, wobei das bereits vermessene Modell in der gleichen Position zum Sensor wie beim ersten Messgang verbleibt Deshalb ist die räumliche Lage des Bissregistrats zum Kiefermodell dem Messsystem (Computer) bekannt. Die räumliche Lage wird beispielsweise entweder auf Grund der Repositionierung des Kiefers und/oder die Repositionierung durch einen softwaretechnischen Vorgang (Matching) der Kiefermodelle vom 1. Scanvorgang und 2. Scanvorgang erreicht.

Das Computerprogramm handhabt die zweite Messung jedoch so, dass das Bissregistrat zum Zweck der Modellierung auf dem Bildschirm ein- oder ausgeblendet werden kann. Alternativ kann das Registrat kann auch transparent dargestellt werden.

### 3. Stabgeschiebe / Schröderzapfen

Ausgangsbasis bei diesem Aspekt der Erfindung ist ferner, dass Daten eines mit einem Stabgeschiebe zu versehenden 3D-Datensatz eines Zahnersatzteils (z.B. Krone oder Brücke) vorliegen. Bei diesem Aspekt der Erfindung handelt es sich um eine Verbesserung bisher in der Zahntechnik üblicher so genannter Stabgeschiebe oder Schröderzapfen.

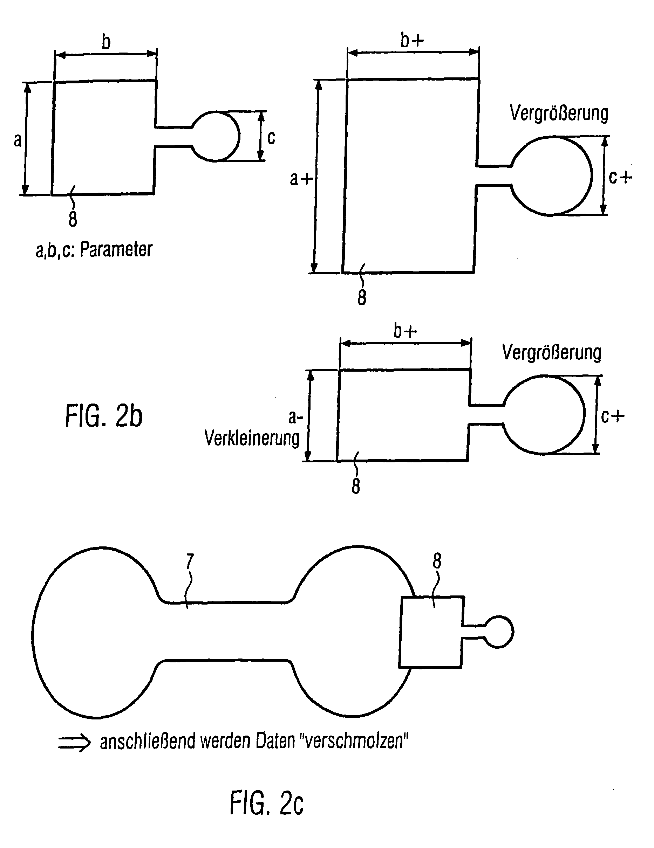

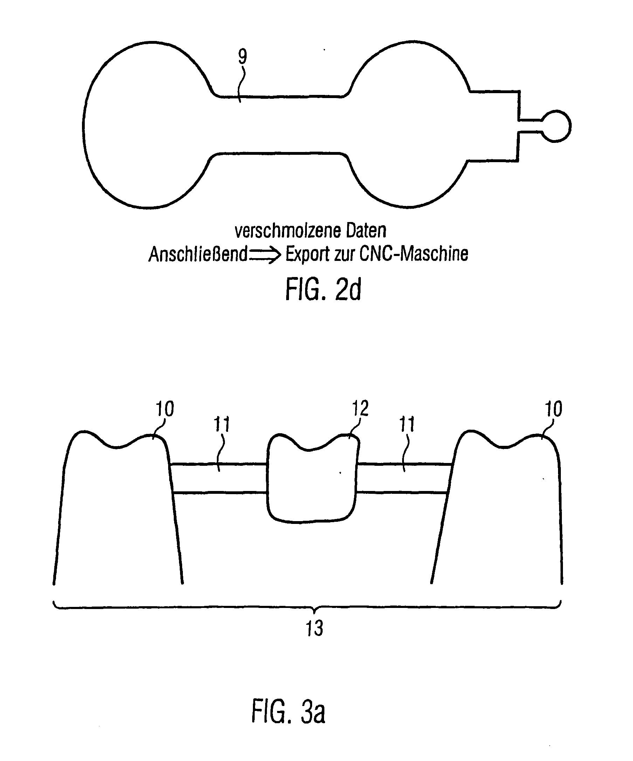

Geometrisch vordefinierte Körper (siehe oben) können mit oder per Software zu den vorliegenden Daten hinzugefügt (importiert) werden. Anschließend können die Daten softwaretechnisch so "verschmolzen" werden, dass sich ein Datensatz oder eine Kombination von Datensätzen ergibt, die zur Fertigung per CNC-Maschine weiterverarbeitet werden können. Dementsprechend werden in diesem zahntechnischen Anwendungsfall die geometrischen Abmessungen eines Stabgeschiebes oder Schröderzapfens zum bestehenden Zahnersatzdatensatz importiert und auf einem Bildschirm dargestellt (Fig. 2a). Anschließend wird der Zapfen zum Zahnersatzteil z.B. per Mausfunktion räumlich angeordnet und mit dem Zahnersatzteil, z.B. Brückengerüst, softwaretechnisch verschmolzen (Fig. 2d) oder deren räumliche Lage zueinander kann abgespeichert werden. Die Geometriedaten können in parametrischer Form importiert werden, d.h.: z.B. können Geometrieabmessungen per Maus gedehnt oder gestaucht werden, während Längenskalen am Bildschirm eingeblendet sind (Fig. 2b, 2c).

Insbesondere eignet sich ein Stabgeschiebe (Matrize) zum Verbinden von zwei (Teil-) Brücken, welche auf Grund der zahntechnischen Geometrien und Anordnungen zwei unterschiedliche Haupteinschubrichtungen benötigen. Dies ist besser bekannt als "Teilungsgeschiebe", wobei die Z-Richtung des Stabgeschiebes insbesondere aus der Haupteinschubrichtung der zweiten Brücke genommen wird. Somit ist sichergestellt, dass über eine einfache Möglichkeit das Stabgeschiebe als Verbinder der zwei Brücken verwendet werden kann.

Bei einer anderen Weiterbildung wird das Stabgeschiebe in der Funktion "Teilungsgeschiebe" so ausgeführt, dass sich die bereits getätigte Konstruktion/Geometrie dafür eignet, in der zweiten (Teil-) Brücke das Gegenstück (Patrize) zu ermitteln, indem das Stabgeschiebeelement in einer mathematischen Operation, wie z.B. entsprechend einer Booleschen Algebra, von der zweiten Brücke abgezogen wird und dadurch das passende Gegenstück erzeugt wird.

### 4. Erweiterung Ponticdatenbank

Bei diesem Aspekt der Erfindung handelt es sich um eine Verbesserung bisher in der Zahntechnik üblicher so genannter Pontics (Zwischenglieder: Diese Konstruktionselemente hängen frei zwischen Pfeilerzähnen und ersetzten funktional einen kompletten fehlenden Zahn) im bzw. zum Einsatz in der CAD/CAM-Technologie für Zahntechnik

Bisher wird von der Software oftmals ein in einer Datenbank abgespeichertes Pontic als Zwischenglied vorgeschlagen.

In der erfindungsgemäß verbesserten Ausführung wird ein der Position des Zahnes entsprechendes Zwischenglied aus einer Datenbank entnommen. Je nach Geschmack des Zahntechnikers können nun verschiedene Ponticformen aus der Datenbank ausgewählt, entnommen und eingesetzt werden. Vorteilhaft sind Vorschläge mit den Grundformen "konvex", "konkav", "plan" im basalen Bereich von Pontics. Ein Aspekt der Erfindung, der für den allgemein bekannten Stand der Technik Gültigkeit hat, besteht in der Möglichkeit, eine individuelle Ponticdatenbank für den Kunden anzubieten. Hierbei entwirft der Zahntechniker mittels CAD-Technik eigene Pontics, die er in einer individuellen Datenbank hinterlegen und abspeichern kann. Bei einer neuen Zahnsituation kann er das selbst entworfene Pontic aus der Kundendatenbank entnehmen, weiterhin geometrisch ändern und anpassen und in den Datensatz für die Brückenkonstruktion eingliedem.

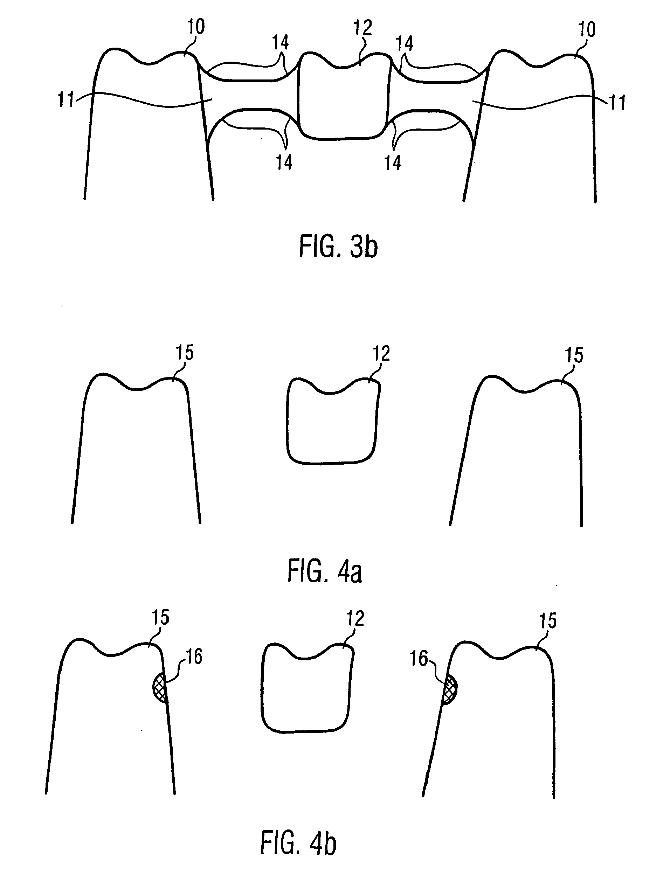

Die Auswahl kann vom Zahntechniker in der Software auch so voreingestellt werden, dass die von ihm favorisierte Form automatisch als Erstvorschlag für die jeweilige Zahnposition erscheint. Weiterhin kann er eine Rangfolge der Favoriten bestimmen und abspeichern. Diese Rangfolge wird nach jedem weiteren Eingabevorgang ersetzt, bis er diesem Vorgang mit einer speziellen Tastenfunktion des Computers, z.B. der "Enter"-Taste bestätigt. Dieselben hier beschriebenen Vorgehensweisen können auch für Verbinder (Fig. 3a, 3b) angewendet werden.

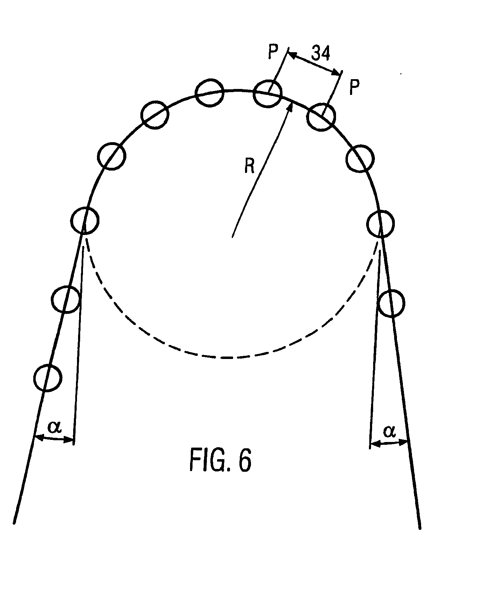

In weiterer Ausgestaltung kann die Bibliothek sozusagen in normierter Größe ausgeführt sein. Wenn ein Zahntechniker ein Pontic auswählt, können über ein Verfahren mit einem Kreis-Tangenten-Modell (vergleiche Figur 6) die Positionen und Skalierungen der Pontics in größeren und kleineren Kiefern aus der programmierten Bibliothek eingefügt werden. Dies erspart einem Anwender sehr viel manuelle Computerarbeit. Lediglich zum besseren Verständnis wird noch angegeben, dass es sich bei dem Kreis-Tangenten-Modell um eine schematisierte Ausführung eines menschlichen Kiefermodells handelt.

### 5. Automatische Formanpassung für den Übergang Verbinder / Krone

Bei diesem Aspekt der Erfindung handelt es sich um eine Verbesserung bisher in der Zahntechnik üblicher so genannter Verbinder bzw. Verbindungsstege zwischen Zahnkronen und Pontics.

Im bisherigen Stand der Technik wurde ein stabförmiger Verbinder gewählt, der verformt werden kann. Der Übergang zwischen Verbinder und Krone war bislang scharfkantig. Dieser ungewünschte Effekt wurde durch CAM-Berechnungen handelsüblicher CAM-Module zur Frästechnik quasi als Abfallprodukt abgeschwächt, nachdem 3D-Fräsbahnberechungen nicht in der Lage sind, solche scharfkantigen Übergänge zu fräsen.

Gemäß der neuen Erfindung wird der Datensatz bereits im Rahmen der Modulationssoftware (CAD-Modul) von einer Software speziell an diesen Stellen untersucht, an denen zwei zu verschmelzende Datensätze (z.B. derjenige einer Krone und derjenige eines Verbinders und derjenige eines Pontics) aufeinander treffen. Je nach Form der aufeinander treffenden Datensätze wird sodann eine automatische Verrundung erzeugt. Die Verrundung kann z.B. durch einen vorgegebenen oder wählbaren Radius erfolgen. Sie kann aber auch vollautomatisch entsprechend der anatomischen Gegebenheiten der speziellen Zahnposition im Kiefer (z.B. Backenzahnbereich, 4.

Zahn links, etc.) erfolgen. Für die jeweilige Position charakteristische Parameter können in einer Datenbank hinterlegt sein, die bei Erkennung der Position automatisch geladen werden. Eine dritte Möglichkeit besteht darin, dass die Software die angrenzenden Umgebungstlächen untersucht und eine möglichst gleichmäßig wirkende Oberflächenanpassung mittels einer so genannten Triangulations-Netzentspannung vornimmt. (Fig. 3a, 3b). Eine weitere Möglichkeit besteht darin, dass komplette Verbinderformen inklusive Verrundungen in einer Datenbank hinterlegt sind (beispielsweise ermittelt durch Abscannen von manuell gefertigten Wachsverbindem).

### 6. Individuelle Abutments

Ausgangsbasis bei diesem Aspekt der Erfindung ist ferner, dass dreidimensionale Daten eines menschlichen Kiefers mit Implantatspfosten vorliegen. Bei diesem Aspekt der Erfindung handelt es sich um eine Verbesserung bisher in der Zahntechnik üblicher so genannter Abutments.

Der bisher bekannte Stand der Technik erlaubt die Verwendung von Abutmentrohlingen, die in mühseliger Handarbeit an die gegeben Zahnsituation angepasst werden müssen.

Die räumliche Lage und Form von Implantatspfosten und ggf. Nachbarzähnen im Kiefermodell wird mittels eines 3D-Scans ermittelt. Dabei kann unter Zuhilfenahme einer bereits ebenfalls durch Scannen bekannten 3D-Geometrie des 3D-Scans auf die exakte Lage des Implantatpfostens rückgeschlossen werden.

Nun wird aus einer Datenbank ein abgespeichertes, für die Position des Zahns im Kiefermodell passendes Abutment entnommen. Diese Abutment ist parametrisch abgespeichert und dessen Form kann aus wie z.B. in Fig. 2b softwaretechnisch verändert werden. Das Abutment wird auf dem Bildschirm auf den Implantatspfosten aufgesetzt und entsprechend an die Situation verändert. Neben den parametrischen Eingabemöglichkeiten (siehe Fig. 2b) können auch Morphingfunktionen zur Oberflächenbearbeitung zur Veränderung des Abutments herangeführt werden.

Die veränderten Daten werden abgespeichert und können an eine CNC-Software zur Herstellung eines individuellen Abutments übergeben werden.

Alternativ kann auf dem Implantatspfosten ein gewünschtes Abutment mit Wachs modelliert werden. Dieses Wachsmodell wird in einem zweiten Scanvorgang eingescannt (Obertlächendaten). Die entweder vorher eingescannten Geometriedaten oder die vorbekannte Geometrie des Implantatpfostens wird per Software mit dem Oberflächendatensatz des Wachsmodells so zusammengeführt, dass sich eine kompletter Formdatensatz eines Abutments ergibt (Ober- und Unterseite zusammengefttgt).

Diese Daten werden an eine CNC-Fertigungssoftware übergeben und anschließend per CNC-Maschine gefräst.

### 7. Marylandbrücken

Ausgangsbasis bei diesem Aspekt der Erfindung ist ferner, dass dreidimensionale Daten eines menschlichen Kiefers mit Zahnlücken vorliegen. Bei diesem Aspekt der Erfindung handelt es sich um eine Verbesserung bisher in der Zahntechnik üblicher so genannter Marylandbrücken.

Ausgangssituation ist eine Zahnlücke zwischen zwei Nachbarzähnen. Die Messdaten dieser Situation liegen vor. Anstelle der Zahnlücke soll eine Brücke zwischen den beiden Nachbarzähnen eingeklebt werden.

Der Zahntechniker markiert auf den an die Brücken angrenzenden Nachbarzähnen die späteren Klebeflächen (z.B. eine auf die Zahnoberfläche projizierte Kreisform) für die Marylandbrücke. Die Marylandbrücke wird im Anschluss basierend auf den Messdaten der Situation unter Einbeziehung der Klebefläche am Bildschirm konstruiert. Nach der Markierung der Klebeflächen wird von der Software ein automatischer Vorschlag generiert (Pontics, Verbinder), der im Anschluss vom Zahntechniker modifiziert werden kann.

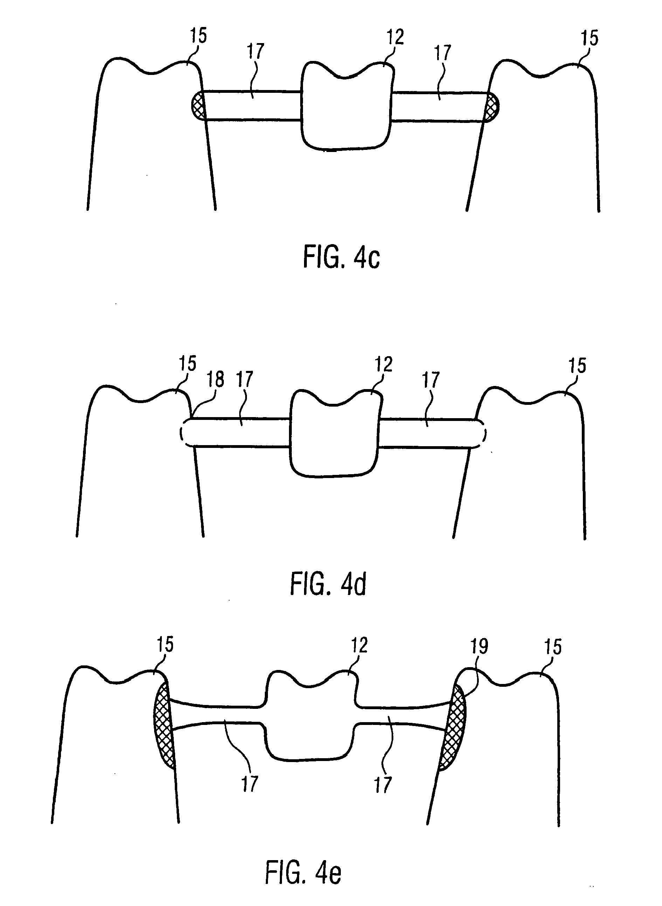

Beispielsweise wird in einem ersten Schritt ein Pontic aus einer Datenbank geladen und an die Stelle der Zahnlücke gebracht (Fig. 4a) - die angegebene Reihenfolge der Verfahrensschritte ist dabei nicht zwingend, sondern kann insbesondere entsprechend anderen Erfordernissen angepasst sein. Im nächsten Schritt wird an den Nachbarzähnen am Bildschirm eine Markierung angebracht, an der sich die Klebestellen für die Marylandbrücken befinden sollen. Die Markierung kann alle möglichen Flächenformen darstellen, z.B. einen projizierten Kreis. Ausgehend von dieser Markierung generiert die Software einen Verbinder (Fig. 4c). Auch an dem Pontic können Markierungen angebracht werden. Die Verbinder werden mit dem Pontic softwaretechnisch verschmolzen. Am Ende des Verbinders können vollautomatisch Verrundungen erzeugt werden. Dies ist bei der Marylandbrücke besonders vorteilhaft, da sich dadurch die Klebefläche und Gesamtstabilität deutlich erhöht (Fig. 4e).

Alternativ können Pontic und Verbinder importiert werden. An der Schnittkante zum Nachbarzahn kann die Software einen "Abschneidevorgang" durchführen, d.h. der Verbinder wird softwaretechnisch abgeschnitten und ggf. anschließend von der Software verrundet.

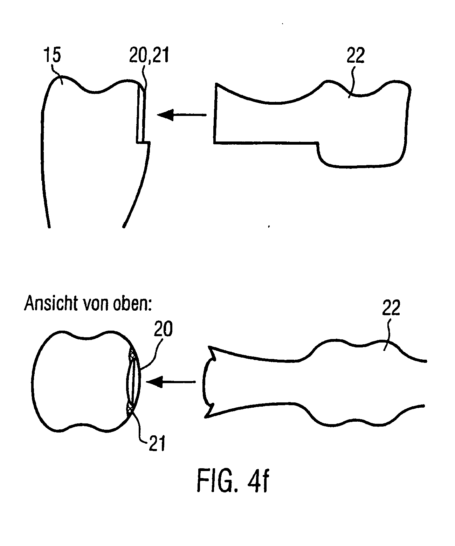

Eine weitere vorteilhafte Ausbildung besteht darin, vor der ersten Vermessung im Nachbarzahn eine oder mehrere Vertiefungsrillen einzubringen, die die Bruchfestigkeit der Maryiandbrückenkonstruktion an der Klebestelle erhöhen, nachdem sich die Rillen als mechanischer Anschlag ausbilden lassen (Fig. 4f).

Nun wird der Datensatz an eine CNC-Maschine zur Fertigung übergeben. Anschließend wird die Brücke eingeklebt.

Weitere Schritte des Verfahrens sowie Komponenten der Vorrichtung für diesen Erfindungsaspekt sind in den Fig. 4b und 4d gezeigt und verdeutlicht.

### 8. Scannen von Aufstellungen

Bei diesem Aspekt der Erfindung handelt es sich um eine Verbesserung bisher in der Zahntechnik üblicher so genannter Aufstellungen bei CAD-Brückenkonstruktionen.

Das Scannen von Aufstellungen dient zur Konstruktionshilfe komplexerer Arbeiten am Bildschirm. Nach der ersten Vermessung werden vom Zahntechniker auf dem Gipsmodell per Wachs (die Firma Etkon AG hat beispielsweise ein spezielles Scanwachs entwickelt) grobe Aufstellungen gemacht, die in einem zweiten Scandurchgang vermessen werden. Die zwei Scans könne auch über ein mathematisches Verfahren (Matching) hochgenau zueinander korreliert werden. Die Aufstellungen können zur Konstruktionshilfe am Bildschirm ein- oder ausgeblendet bzw. transparent dargestellt werden. Nachdem die Aufstellung nur grob (zur groben Orientierung) erfolgt, entsteht kein großer Zeitaufwand für den Zahntechniker (oftmals ist auch bereits ein Aufstellung vorhanden).

### 9. Farbliche Wandstärkenkontrollen

Bei diesem Aspekt der Erfindung handelt es sich um eine Verbesserung bisher in der Zahntechnik üblicher so genannter Wandstärkenkontrollen.

Während der Modellation oder Modellierung am Bildschirm kann der Zahntechniker die Veränderungen der Wandstärken dadurch beurteilen, dass die lokalen Wandstärken der Restauration am Bildschirm in farblichen Abstufungen gekennzeichnet dargestellt werden. Auch eine transparente Darstellung ist möglich.

### 10. Verfahren zur Herstellung von Inlays mittels Copy-Cad-Funktion

Bei diesem Aspekt der Erfindung handelt es sich um eine Verbesserung bisher in der Zahntechnik üblicher so genannter Inlays.

Herstellungsverfahren für Inlays.

Die Kavität wird gescannt. Anschließend wird eine Kantendetektion durchgeführt. Danach wird das Inlay im Modell mit Wachs modelliert und in diesem Zustand nochmals gescannt. Die beiden Messdatensätze werden per 3D-Matching überlagert. Abschließend ein Differenzdatensatz errechnet (Dies ist das Volumen des Inlays). Dabei ist die neue Besonderheit, dass die vorab durchgeführte Kantendetektion zur Festlegung der Grenze (aus Präzisionsgründen) herangezogen wird.

Es kann auch auf eine explizite Kantendetektion verzichtet werden, und es können über ein kombiniertes Verfahren der "Korrelation" (Übereinanderlegung von zwei Scans) und der "Abstandsdetektion" der zwei Scans diejenigen Flächen/Punkte herausgefiltert werden, welche für ein Inlay relevant sind (oder anders ausgedrückt, ein Verfahren zur Volumendifferenzbildung wird angewandt). Ein wesentlicher Vorteil dieser Variante ist der damit mögliche vollautomatisierte Betrieb.

### 11. 3D-Detektion der räumlichen Lage von Implantatspfosten im Patientenmund

Bei diesem Aspekt der Erfindung handelt es sich um eine Verbesserung bisher in der Zahntechnik üblicher so genannter Implantatspfostentechnologie.

In einem ersten Schritt wird mittels Einscannen einer Gipsmodellsituation die räumliche Lage der Implantatspfosten bzw. der Implantatsschrauben bestimmt, wobei dies auch über so genannte "Dummy-Implantate" geschehen kann, deren Geometrie bereits bekannt ist, und die sich dadurch auszeichnen, dass sich diese Dummy-Körper besser vermessen lassen und sich auch besser eigenen, damit sich über mathematische Operationen die räumlichen Lagen der Implantate bestimmen lassen. In einem zweiten Schritt werden die Geometriedaten der Implantatspfosten (gemäß Herstellerangaben) importiert. Die importierten Daten werden mit den bei der 3D-Vermessung ermittelten Daten entweder gematcht und am Bildschirm angezeigt. Durch den Import der Solldaten wird die Präzision erhöht. In einem nächsten Schritt werden Kronen- und Brückenkonstruktionen auf Basis der bisher beschriebenen Softwareschritte am Bildschirm konstruiert und an die CNC-Maschine zur Fertigung übermittelt.

### 12. Verfahren zur Herstellung von Primärkronen

Bei diesem Aspekt der Erfindung handelt es sich um eine Verbesserung bisher in der Zahntechnik üblicher so genannter Primärkronen.

Eine Softwaregenerierte Primärkrone aus dem aus den Anmeldungen der Firma Willytec GmbH bekannten Stand der Technik weist an der Außenseite glatte Flächen auf, die aus der in Fig. 5d, gezeigten Ansicht bezogen auf die Einschubrichtung parallel oder konisch verlaufen können (Fig. 5d, Winkel Alpha wäre ein Konuswinkel). Je nach Lage der Flächen ergibt sich eine neue Topographie der Primärkrone (z.B. durch Veränderung des Maßes A, Fig. 5d). Erfindungsgemäß können per Software eine Reihe von Veränderungen an der Geometrie der Primärkrone verändert werden. Beispielsweise können Wandstärkenparameter das Maß A in der Fig. 5d verändern, oder die Eingabe von Konuswinkeln (Alpha).

Wesentlich für die Ausgestaltung von Primärkronen aus Sicht des Zahntechnikers ist jedoch die Bearbeitung der Friktionslinien (Fig. 5d). Die Erfindung sieht vor, dass der Zahntechniker eine sich geometrisch vorhandene und auf dem Bildschirm sichtbare Friktionslinie zuerst selektieren kann. Sodann kann er eine Stelle der Friktionslinie (Punkt) markieren. Ausgehend von diesem Punkt kann er einen Wirkungsbereich gemäß Fig. 5d wählen, also die Breite der zu verändernden Stelle. Im nächsten Schritt kann der die Friktionslinie an diesem Punkt über den gewählten Wirkungsbereich nach oben, unten, innen oder außen ziehen (Fig. 5e). Daraus ergibt sich eine neue Topographie der Primärkrone unter automatischer Beibehaltung (softwaregesteuert) der Parallelität zur Einschubrichtung bzw. unter Beibehaltung des Konuswinkels.

Gleiches gilt für beide Friktionslinien. Zieht der Zahntechniker beispielsweise die Friktionslinie nach außen, so ist die Software erfindungsgemäß so beschaffen, dass die jeweils korrespondierende Friktionslinie mitgezogen wird. Dies ist zur Beibehaltung der Parallelität bzw. des Konuswinkels erforderlich.

Eine weitere Erfindung befasst sich mit Fertigung von Primärkronen insbesondere mittels so genannter 3+1 (angestellt) Achsenftästechnik.

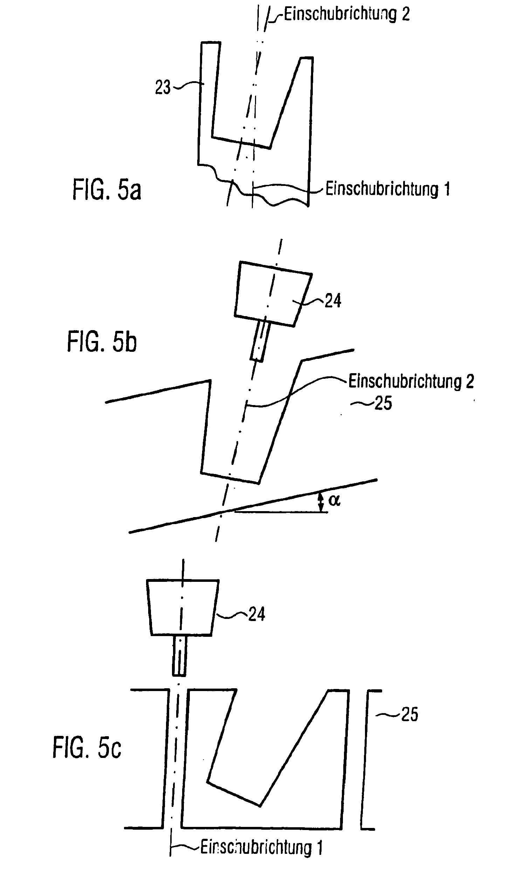

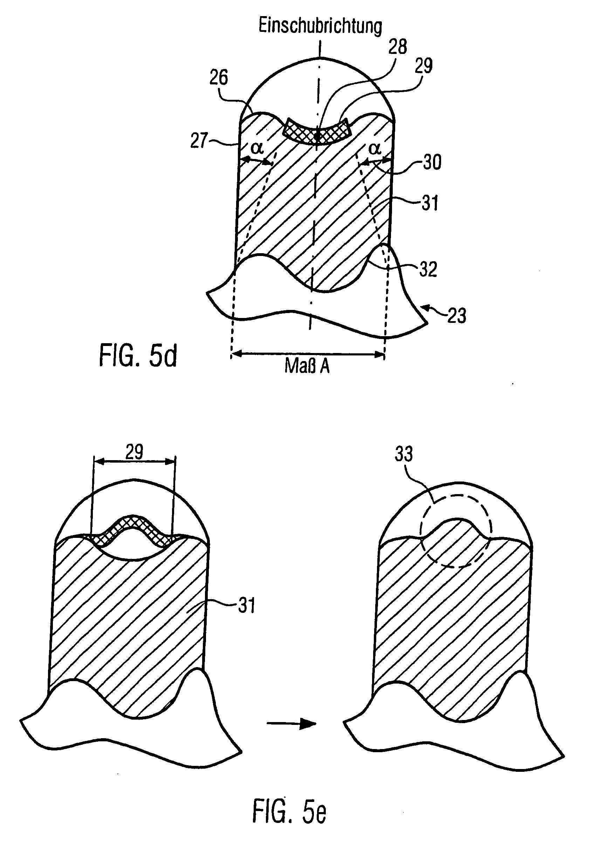

Nachdem Primärkronen zwei Einschubrichtungen aufweisen, ist es besonders vorteilhaft, den Fräser beim Fräsvorgang exakt gemäß der Einschubrichtung auszurichten (Fig. 5a bis 5d). Beim erfindungsgemäßen Verfahren erfolgt dies durch Verkippung des Fräsrohlings oder des Fräsers gemäß der Winkeldifferenz der beiden Einschubrichtungen. In einem ersten Fräsdurchgang wird der Fräser parallel zur Einschubrichtung 1 ausgerichtet. In dieser Ausrichtung bearbeitet er alle Daten, die sich auf die Einschubrichtung 1 beziehen. Anschließend wird das Werkstück bzw. der Fräser gemäß Einschubrichtung 2 ausgerichtet und er bearbeitet alle Daten, die sich auf die Einschubrichtung 2 beziehen (Fig. 5a bis 5d). Durch diese Frässtrategie können besonders gute O-berflächenqualitäten und Passungen an all jenen Flächen generiert werden, die parallel zu Einschubrichtungen liegen. Eine besondere Bedeutung kommt hierbei die separate Einstellung der Stumpfeinschubrichtung (Einschubrichtung 2) in nur "4 Achsen" zu, da dadurch auf die sehr teuere 5-Achsen-Technik verzichtet werden kann. Hierbei wird das ganze Modell in einer Art und weise um die Z-Achse gedreht, damit eventuell vorhandene Unterschnitte in genau der 4. Achse ausgeschlossen werden können. Dadurch kann auf eine recht teuere 5. Achse verzichtet werden. Dies ist für eine erfolgreiche Verwendung der Primärkronentechnik von hoher Bedeutung.

Weitere Einzelheiten ergeben sich aus der Fig. 5e.

### 13. Fräsen von Unterschnitten in Brückenkonstruktionen :

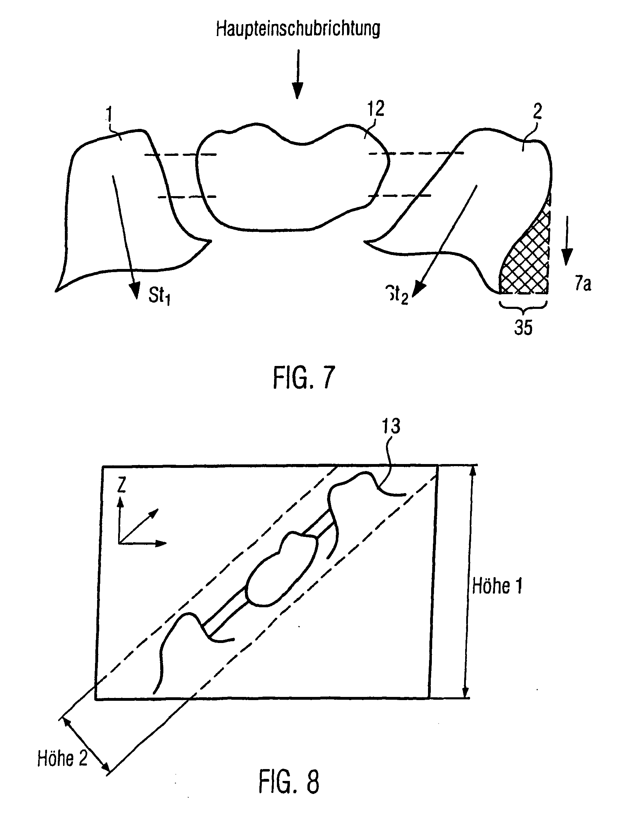

Es kommt in der Zahntechnik häufiger vor, dass keine Haupteinschubrichtung für eine mehrgliedrige Brücke erstellt werden kann, ohne zu starke Unterschnitte in einzelnen Stümpfen zu erzeugen, wie in der Fig. 7 verdeutlicht ist (siehe Detail 7a). Eine Hinterschnittskorrektur, wie im Zusammenhang mit dem entsprechend den Ausführungsbeispielen durch eine Software realisierten Verfahren, kann eben geeignete Korrekturen, wie z.B. Entfernen der Unterschnitte in einzelnen Stümpfen, erzielen, hat aber eventuell den Nachteil, dass dadurch abstehende Ränder erzeugt werden, wie in der Fig. 7 verdeutlicht ist (siehe Detail 7a).

Eine erfindungsgemäße Lösung besteht darin, dass durch ein entsprechendes Verfahren die vorstehenden Nachteile minimiert oder ganz behoben werden. Es wird verfahrensgemäß z.B. durch eine Software eine zusätzliche Einschubrichtung St₂ ermittelt und an eine Fräsmaschine weitergegeben. Weiter kann durch verfahrensmäßige, wie beispielsweise softwaretechnische Begrenzungen verhindert werden, dass eine solche Brücke nicht mehr einsetzbar wird.

zusätzlich kann mit der vorstehenden Methodik analog zur weiter oben beschriebenen Primärkronentechnik durch ein entsprechendes Verfahren die Richtung des St₂-Stumpfes so gewählt werden, dass anstelle einer 5-Achsen-Maschine für die Bearbeitung nur eine 4-Achsen-Maschine erforderlich ist.

### 14. Erweiterung der Primärkronen (Primärkonstruktionen), Sekundärkonstruktion und Tertiärkonstruktion

In der Zahntechnik ist es üblich, auf den Primärkonstruktionen direkt eine Brückenkonstruktion (Sekundärkonstruktion) oder über den Zwischenschritt von z.B. "Galvanosekundärteilen" eine herausnehmbare oder festsitzende Tertiärkonstruktion zu erstellen. Das hier vorgestellte Verfahren erleichtert diesen Prozess ungemein, da die geometrischen Daten der Primärteile bereits gespeichert sind und auf diesen über spezielle Verfahrens- oder insbesondere Softwarefunktionen in einfachster Weise eine Brückenkonstruktion erstellt werden kann.

### 15. "Bergauf-/Bergabfräsen"

Das "Bergauf-/Bergabfräsen" zeichnet sich durch eine optimierte Ausrichtung eines Fräsrohlings zu dem zu fräsenden Teil aus. Bei mehrgliedrigen Brücken ist oft, wie in der Fig. 8 schematisch veranschaulicht ist, erforderlich, einen deutlich dickeren Rohling zu verwenden, da in 3-Achsen-Maschinen die Bearbeitung der Rohlinge nur in zwei Richtungen möglich ist. Durch die Berechnung des Mindestvolumens in der richtigen Richtung und Transformation der Brücke im Raum ist es möglich, einen deutlich kleineren Rohling für das Fräsen der Brücke zu verwenden. Verdeutlicht ist dies durch den Vergleich von einerseits der Höhe 1 und andererseits der optimierten Höhe 2 in der Fig. 8. Somit ist es mit einer 5-Achsen-Maschine möglich, eine deutlich optimiertere Frässtrategie anzuwenden.

### 16. "Copy-Cad-Wachs"

Ein sehr verbreitetes Problem ist das Scannen von Modellierwachsen, da sich die teildurchlässige Struktur von Wachs schlecht oder gar nicht scannen lässt. Speziell durch die Zugabe von mindestens 1/3 bis 4/5 Gewichtsanteilen von Zirkonoxidpulver wird das Wachs sehr gut scanbar, ohne dabei die typischen Modelliereigenschaften zu verlieren. Ein solcher Effekt wurde bei der Zugabe von anderen Stoffen nicht beobachtet.

Nachfolgend werden noch weitere Ausführungsmöglichkeiten und Erweiterungen für die einzelnen vorstehend erläuterten Ausführungsbeispiele angegeben.

Um das Arbeiten mit den ermittelten Daten z.B. eines Zahnersatzteils zu erleichtern, kann vorgesehen sein, dass eine farbige Darstellung von Oberflächenbereichen erfolgt. Dazu werden die Daten oder ein daraus erzeugtes elektronisches Bild, wie beispielsweise an einem Computerbildschirm, entweder frei wählbar, manuell oder automatisch anhand von vorgegebenen Kriterien in Oberflächenbereiche eingeteilt. Dann werden diesen Einteilungen und/oder Oberflächenbereichen einzelne Farbgrenzen bzw. Farben zugeordnet und es erfolgt eine entsprechende Darstellung.

So zum Bespiel kann die Innenseite des Zahnersatzteils einem bestimmten Bereich zugeordnet sein. Die Einteilung hat den Vorteil, dass für bestimmte Bereiche spezielle Fertigungsinformationen hinterlegt werden können. Beispielsweise soll auf der Innenseite eines Zahnersatzteiles von einer Maschine genauer gefertigt werden als auf der Außenseite. Besonders präzise soll im Bereich der Präparationsgrenze gearbeitet werden. Hierfür muss z.B. von einer Maschine mit kleinem Werkzeug, hoher Drehzahl und kleinen Vorschüben gearbeitet werden.

Die Einteilung in solche Bereiche wird nach der Erzeugung der Formdatensätze in einem allgemeingültigen Datenformat hinterlegt, das von der Fertigungsart (z.B. Fräsen, Lasersintering, etc.) oder Fertigungsmaschine unabhängig ist. Als Beispiel für ein solches Format könnte ein STL-Format mit Ergänzungsinformationen verwendet werden. Dieses Format kann an verschiedene weiterverarbeitende Systeme (CAM) übermittelt werden.

Soweit vorstehend von einer Software oder ihrer Spezifizierung, Gestaltung, Umsetzung und Anwendung gesprochen wird, ist dies immer als eine Realisierung der Erfindung im Umfang eines entsprechenden Ausführungsbeispiels sowohl als Verfahren, als auch durch entsprechend geeignete und erforderliche Vorrichtungen und Einrichtungen zu verstehen. Grundsätzlich sind mit den entsprechenden Angaben für einen Fachmann auch alle anderen möglichen Verfahrensrealisierungen und insbesondere Automatisierungen klar. Sicherheitshalber wird jedoch hier darauf hingewiesen, dass sämtliche Angaben, die sich auf eine Software beziehen, allgemein als auf ein Verfahren und/oder Einrichtungen bezogen zu verstehen sind.

Die Erfindung ist anhand der Ausführungsbeispiele in der Beschreibung und in den Zeichnungen lediglich exemplarisch dargestellt und nicht darauf beschränkt, sondern umfasst alle Variationen, Modifikationen, Substitutionen und Kombinationen, die der Fachmann den vorliegenden Unterlagen insbesondere im Rahmen der Ansprüche und der allgemeinen Darstellungen in der Einleitung dieser Beschreibung sowie der Beschreibung der Ausführungsbeispiele und deren Darstellungen in der Zeichnung entnehmen und mit seinem fachmännischen Wissen sowie dem Stand der Technik kombinieren kann. Insbesondere sind alle einzelnen Merkmale und Ausgestaltungsmöglichkeiten der Erfindung und ihrer Ausführungsbeispiele kombinierbar.

In den Figuren der Zeichnung bezeichnen:
- 1: Zahnstumpf 1
- 2: Zahnstumpf 2
- 3: Steg
- 4: fräsbares Werkstück
- 5: Zahnsituation (hier gestrichelt)
- 6: abnehmbare Sekundär/Tertiärkonstruktion als Beispiel
- 7: Datensatz Zahnbrücke
- 8: Datensatz geometrisch definierter Zapfen
- 9: verschmolzene Daten aus Datensatz Zahnbrücke und Datensatz geometrisch definierter Zapfen
- 10: Krone
- 11: Verbinder (z.B. stabförmig)
- 12: Pontic
- 13: Brücke
- 14: Verrundungen
- 15: Nachbarzahn
- 16: Markierungen
- 17: Zwischenglied
- 18: Schnittkante
- 19: Verrundung mit größerer Klebefläche
- 20: Rille
- 21: Klebestelle
- 22: Marylandbrücke
- 23: Primärkrone
- 24: Fräser
- 25: Materialrohling
- 26: Friktionslinie oben
- 27: Fläche parallel zur Einschubrichtung
- 28: markierter Punkt
- 29: Wirkungsbereich
- 30: gedachter Konuswinkel α
- 31: glatte Fläche
- 32: Friktionslinie unten
- 33: Ergebnis
- 34: Abstand
- 35: Randschluss

## Patentansprüche

1. Verfahren zur Herstellung von Zahnersatzteilen mit
digitaler Erfassung von Obertlächendaten eines Restzahnbereichs,
dreidimensionaler Gestaltung wenigstens eines Formteils, insbesondere eines Zahnersatzteils für den Restzahnbereich unter Einbeziehung der erfassten Oberflächendaten des Restzahnbereichs und Erstellung entsprechender Formdaten, und
teilweiser oder kompletter Herstellung des wenigstens einen Zahnersatzteiles auf der Basis der Oberflächendaten und Formdaten,
**dadurch gekennzeichnet,**
**dass** nach einer ersten Vermessung auf dem Gipsmodell per Wachs grobe Aufstellungen gemacht werden,
**dass** die groben Aufstellungen in einem zweiten Scandurchgang vermessen werden, und dass die zwei Scans hochgenau zueinander korreliert werden.

2. Verfahren zur Herstellung von Zahnersatzteilen nach Anspruch 1, **dadurch gekennzeichnet, dass** das Korrelieren über ein mathematisches Verfahren, insbesondere Matching, erfolgt.

3. Verfahren zur Herstellung von Zahnersatzteilen nach Anspruch 2, **dadurch gekennzeichnet, dass** die Daten der Aufstellungen zur Konstruktionshilfe am Bildschirm ein- und ausgeblendet oder transparent dargestellt werden.

4. Vorrichtung zur Herstellung von Zahnersatzteilen mit
Einrichtungen zur digitalen Erfassung von Oberflächendaten eines Restzähnbereichs,
Einrichtungen zur dreidimensionalen Gestaltung wenigstens eines Formteils, insbesondere eines Zahnersatzteils für den Restzahnbereich unter Einbeziehung der erfassten Oberflächendaten des Restzahnbereichs und Erstellung entsprechender Formdaten, und Einrichtungen zur teilweisen oder kompletten Herstellung des wenigstens einen Zahnersatzteiles auf der Basis der Oberflächendaten und Formdaten,
**dadurch gekennzeichnet,**
**dass** Einrichtungen zur Durchführung der Verfahren gemäß jeglichem der Ansprüche 1 bis 3, einschließlich Datenverarbeitungseinrichtungen, Datenspeichereinrichtungen, Bildschirmeinrichtungen, Eingabe- und Ausgabeeinrichtungen sowie Datenübertragungseinrichtungen und Materialbearbeitungseinrichtungen vorgesehen sind, zur Durchführung der Verfahren gemäß jeglichem der Ansprüche 1 bis 3 ausgelegt und funktional miteinander gekoppelt sind.
